Europäisches Patentamt

European Patent Office

Office européen des brevets

(11) Numéro de publication : **0 352 190 B1**

(12) **FASCICULE DE BREVET EUROPEEN**

(45) Date de publication du fascicule du brevet :
**11.11.92 Bulletin 92/46**

(51) Int. Cl.⁵ : **A61K 9/14,** A61K 9/50

(21) Numéro de dépôt : **89402061.9**

(22) Date de dépôt : **20.07.89**

(54) **Nouvelle forme unitaire, solide et poreuse comprenant des microparticules et/ou nanoparticules ainsi que sa préparation.**

Le dossier contient des informations techniques présentées postérieurement au dépôt de la demande et ne figurant pas dans le présent fascicule.

(30) Priorité : **21.07.88 FR 8809864**

(43) Date de publication de la demande :
**24.01.90 Bulletin 90/04**

(45) Mention de la délivrance du brevet :
**11.11.92 Bulletin 92/46**

(84) Etats contractants désignés :
**AT BE CH DE ES FR GB GR IT LI LU NL SE**

(56) Documents cités :
**EP-A- 0 284 249**
**WO-A-86/06626**
**DE-A- 1 916 084**
**FR-A- 2 036 890**
**FR-A- 2 613 223**
**GB-A- 2 192 128**
**US-A- 4 693 912**
**CHEMICAL ABSTRACTS, vol. 94, no. 24, 15 juin 1981, page 346, abstract no. 197440p, Columbus, Ohio, US**

(73) Titulaire : **FARMALYOC**
**5 Avenue Charles De Martigny**
**F-94701 Maisons Alfort (FR)**

(72) Inventeur : **Courteille,Frédéric**
**4 Avenue Cousin de Méricourt**
**F-92430 Cachan (FR)**
Inventeur : **Coutel, Anne**
**3 Avenue de la Résidence**
**F-92160 Antony (FR)**
Inventeur : **Lebreton, Guy**
**21 Résidence de la Courcelle**
**F-91190 Gif sur Yvette (FR)**
Inventeur : **Veillard, Michel**
**12 Rue du Docteur Roux**
**F-92330 Sceaux (FR)**

(74) Mandataire : **Savina, Jacques et al**
**RHONE-POULENC SANTE, Service Brevets,**
**20 Avenue Raymond Aron**
**F-92165 Antony Cédex (FR)**

## Description

La présente invention concerne une nouvelle composition constituée d'une forme unitaire, solide et poreuse, comprenant des microparticules ou des nanoparticules ainsi que son procédé de préparation.

La préparation de microparticules et de nanoparticules est principalement utilisée de manière à retarder la dissolution des principes actifs et trouve, de ce fait, de nombreuses applications dans le domaine des médicaments à libération contrôlée ainsi que dans le domaine du masquage du goût des médicaments destinés à l'administration orale. Il a néanmoins toujours été difficile de mettre au point une formulation contenant des microparticules ou des nanoparticules sous forme de doses unitaires et notamment une formulation convenable à une administration orale.

En effet, la préparation industrielle de comprimés et de gélules nécessite des qualités d'écoulement et/ou de cohésion du granulé à diviser que ne possèdent pas nécessairement les microparticules et/ou les nanoparticules.

Le comprimé pose des problèmes d'intégrité des microparticules et/ou des nanoparticules sous l'effet de la compression ; sa vitesse de désagrégation lente ne permet pas toujours de l'administrer après désagrégation et mise en suspension dans un verre d'eau.

La gélule ne permet pas toujours la dispersion des particules dans le tractus gastrointestinal.

De plus, la gélule ainsi que le comprimé posent des problèmes de déglutition particulièrement marqués chez l'enfant et chez le vieillard.

Enfin, le sachet est une forme pharmaceutique coûteuse, d'utilisation non ambulatoire et la forme sirop sec n'est pas souvent réalisable pour des raisons de libération précoce du principe actif hors des microparticules et/ou nanoparticules ou de stabilité physique et/ou chimique de la préparation.

La demande de brevet WO 86 06626 décrit la préparation d'une composition destinée à être mise en sachets, comprenant des microcapsules et des excipients sous forme de granulés pouvant être dissous facilement au moment de l'emploi.

Les brevets français 2 036 890 et 2 366 835 décrivent des formes pharmaceutiques ayant pour caractéristique de se dissoudre ou de se désintégrer rapidement en milieu aqueux ou dans la salive.

Il a été maintenant trouvé que des techniques ayant des objectifs totalement opposés, comme le retard de la dissolution d'un principe actif d'une part et le fait que la forme pharmaceutique se désagrège ou se dissolve rapidement, d'autre part, pouvaient être associées de manière à obtenir une forme unitaire lyophilisée, facilement et rapidement désintégrable dans l'eau et contenant des microparticules et/ou des nanoparticules, et dans laquelle la décantation et/ou la remontée en surface des particules au cours de la lyophilisation a été évitée.

Selon l'invention, le procédé de préparation de la nouvelle forme unitaire, solide consiste à

1) préparer un mélange de micro (et/ou nano) particules contenant une quantité prédéterminée d'un ou plusieurs principes actifs,

2) incorporer le mélange obtenu dans une pâte destinée à être lyophilisée et contenant

    a) au moins une substance choisie parmi

        - des matières épaississantes et structurantes servant de support et

        - des ballasts,

    b) un ou plusieurs agents stabilisants empêchant la décantation et/ou la remontée en surface des micro (et/ou nano) particules dans le mélange, choisis parmi des substances particulaires de taille micronique, inertes vis à vis du mélange et dont la vitesse de sédimentation est du même ordre de grandeur que celle des particules,

    c) éventuellement un ou plusieurs autres principes actifs ou mélanges de micro (et/ou nano) particules contenant un principe actif,

    d) une quantité d'eau convenable de manière à ajuster la viscosité de la composition,

3) lyophiliser la pâte obtenue.

Le produit lyophilisé obtenu peut être divisé mécaniquement en doses unitaires présentant une forme et un volume bien définis, mais il est préférable de répartir la pâte dans des alvéoles de forme et de dimension prédéterminées, préalablement à l'opération de lyophilisation. Le dosage du/des principe(s) actif(s) dans la pâte et la forme et les dimensions des alvéoles étant calculés de manière à obtenir une quantité précisément définie du/des principe(s) actif(s) dans chaque dose unitaire.

Dans la description ci-dessus, ainsi que dans ce qui suivra, on entend par "principe actif" toute substance contenant au moins un produit thérapeutiquement actif, un agent de nutrition, un agent de diagnostic ou un agent cosmétique, celui-ci pouvant être en association avec une ou plusieurs autres substances du même type ou en association avec d'autres micro (ou nano) particules contenant elles-mêmes d'autres substances citées ci-dessus.

Selon l'invention, on appelle microparticule toute particule de diamètre compris entre 1µm et 2 mm. Ces microparticules peuvent être des microsphères, des extrudats, des microcapsules ou des microgranules permettant la rétention d'un principe actif et évitant ainsi sa mise à disponibilité complète et immédiate par simple mise en contact avec les milieux liquides aqueux. On appelle nanoparticule toute particule de diamètre inférieur à 1µm. Ces nano-particules peuvent être des nanosphères ou des nanocapsules et présentent des caractéristiques de rétention du principe actif de même nature que les microparticules, étant entendu que, compte tenu de leur faible dimension, elles sont susceptibles de permettre le passage transépithélial de la muqueuse intestinale et devront, de ce fait, être constituées de préférence de polymères biorésorbables.

Le mélange de micro (ou nano) particules est préparé par toute méthode connue et faisant intervenir l'usage d'un polymère ou d'une substance macromoléculaire. Plus particulièrement, peuvent être utilisées les techniques de microencapsulation par évaporation de solvant, microencapsulation par coacervation, microencapsulation par pelliculage en turbine ou par montage et pelliculage en turbine, extrusion simple, extrusion-sphéronisation, extrusion-sphéronisation et enrobage ou enrobage en lit d'air fluidisé.

La réalisation de ces méthodes est mentionnée plus en détail dans les références données ci-dessous à titre d'exemple :
- demande de brevet français 2 484 281
- T.M. SERAJUDDIN et coll., J. Pharm. Sci., 73 (9), 1203 (1984)
- L. LUZZI et coll., Biochemical Applications of Microencapsulation, CRC Press, Franklin Lim Editor (1984), chap. 1, pages 1 à 19
- A.-C. VIAL-BERNASCONI et coll., S.T.P. Pharma, 4 (5), 397 (1988)
- demande de brevet européen EP 204 596
- F. BRIQUET et coll., S.T.P. Pharma, 2 (22), 986 (1986)
- N. SARISUTA et coll., Drug development and industrial pharmacy, 14 (5), 683 (1988)
- demande de brevet européen EP 193 208
- demande de brevet français FR 2 608 988
- Encyclopedia of polymer science and engineering, vol. 9, 2nd ed., John WILEY and sons inc. (1987), pages 724 à 745
- Theory and Practice of Industrial Pharmacy, 2nd ed., L. LACHMAN, H.A. LIEBERMAN and J.L. KANIG, Ed. Lea et Febiger, Philadelphie (1976), pages 420 à 465
- Formes Pharmaceutiques Nouvelles - Aspects Technologique, Biopharmaceutique et Médical, LAVOISIER Tech. et Doc., P. BURI, F. PUISIEUX, E. DOELKER et J.P. BENOIT, Chap. XV, page 613 (1985).

Il est entendu que les polymères ou les substances macromoléculaires pouvant être employés ne sont pas limités à ceux qui sont cités dans les méthodes ci-dessus. D'autres polymères ou substances macromoléculaires, le cas échéant pharmaceutiquement acceptables, peuvent également être utilisés. A titre d'exemple peuvent être notamment utilisés les dérivés de la cellulose (cellulose, carboxyméthylcellulose, acetophtalate de celulose, méthylcellulose, éthylcellulose, hydroxyethylcellulose, hydroxypropylcellulose, hydroxypropylméthylcellulose, hydroxyméthylcellulose phtalate par exemple), dérivés acryliques (polymétacrylate, polyacrylamide, polyacryldextran, polyalkylcyanoacrylate par exemple), acide alginique, dextran, gélatine, polyéthylène glycol, alcool polyvinylique, alginate de propylèneglycol, amidon, zeine, saccharose, polyacétal, acide polylactique polyglycolique, acide polyhydroxybutyrique, polyamide, polyanhydride, poly ε caprolactone, polydiméthylsiloxane, polyorganophosphazene, polyorthoesters, polyaminoacides, polyvinylpyrrolidone, chitine et dérivés, collagène, polyglutaraldéhyde, polypropylène, polytétrafluoroéthylène, polyéthylène, chlorure de polyvinyle, polyuréthanes.

On entend par matière épaississante et structurante servant de support, toute matière soluble ou dispersible dans l'eau, permettant d'assurer la cohésion de la masse, le cas échéant acceptable du point de vue pharmaceutique et inerte vis-à-vis du principe actif. Ces matières sont notamment choisies parmi les polypeptides comme la gélatine ou la gélatine partiellement hydrolysée, des colloïdes, des polysaccharides à poids moléculaire élevé, des hauts polymères pouvant donner des solutions colloïdales, par exemple des gommes naturelles (gomme arabique, gomme adragante...), des gommes synthétiques ou hémisynthétiques (glycosylglucanes, gomme xanthane...), le dextrane, la dextrine, les alginates (alginate de sodium), les pectinates, les dérivés de la cellulose (cellulose microcristalline, carboxyméthylcellulose...), les dérivés hydrodispersibles de l'amidon, les silices colloïdales, les bentonites, ou encore d'autres matières support telles que l'alcool polyvinylique, la polyvinylpyrrolidone, les polyéthylèneglycols (PEG 20 000, PEG 6 000 notamment), des polymères acryliques ou des copolymères, ou encore des mélanges de matières telles que citées ci-dessus.

De préférence, on utilise une matière hydrosoluble.

On désigne par "ballasts" des matières de préférence solubles et cristallisables et le cas échéant pharmaceutiquement acceptables qui améliorent les propriétés physiques de la nouvelle forme unitaire. Ces substances peuvent être notamment choisies parmi le lactose, le glycocolle, le sorbitol, le mannitol, le glucose, les maltodextrines ou encore, éventuellement parmi des oxydes (oxyde de magnésium), des carbonates (carbonate de calcium), des phosphates (phosphate tricalcique), ou la cellulose micro-cristalline, ou des mélanges de ces substances.

Il est entendu que la pâte destinée à la lyophilisation doit nécessairement contenir au moins une substance choisie parmi les matières épaississantes et les ballasts cités ci-dessus, mais il est également avantageux de faire intervenir à la fois une ou plusieurs matières épaississantes et un ou plusieurs ballasts.

Les matières épaississantes et structurantes et les ballasts sont choisis de manière à conférer à la pâte destinée à la lyophilisation, un comportement rhéologique et une viscosité adaptés à une bonne division du produit et à la tenue en suspension des divers composants (écoulement, homogénéité, régularité du volume divisé, stabilité de la suspension pendant la division). Ces substances sont également choisies de manière à assurer la texture du produit final lyophilisé (par exemple dureté suffisante pour permettre l'extrusion à travers un blister).

La pâte destinée à la lyophilisation doit, en outre, contenir un ou plusieurs agents de suspension destinés à empêcher la sédimentation des micro (ou nano) particules pendant la lyophilisation. Ces agents stabilisants sont des substances particulaires solides de taille micronique, inertes vis-à-vis du mélange. Elles peuvent être soit insolubles, soit solubles et en excès par rapport au pouvoir solubilisant de la pâte à lyophiliser. Elles sont choisies de telle manière que leur vitesse de sédimentation soit du même ordre de grandeur que celles des micro (ou nano) particules. Le choix de l'agent stabilisant est de ce fait adapté en fonction de la densité et de la dimension des micro (ou nano) particules ainsi que des conditions de pH de la solution dans laquelle il est introduit. Parmi les agents stabilisants convenables peuvent être cités des oxydes (oxyde de titane, oxyde de magnésium, oxydes de fer, silice par exemple), des sels comme des carbonates (carbonate de calcium, carbonate de magnésium par exemple), des silicates (kaolin par exemple), des phosphates (phosphate tricalcique par exemple), des sucres (lactose, glucose, mannitol, lévulose, maltodextrine par exemple).

Dans le cas de nanoparticules de densité proche de celle de la phase aqueuse de la pâte à lyophiliser, cet additif peut s'avérer non indispensable.

Dans le procédé selon l'invention, il est entendu que l'ordre d'introduction des différentes substances dépend de ces substances elles-mêmes, certaines d'entre elles pouvant être mélangées préalablement.

D'une manière générale, les matières épaississantes et structurantes constituent de 0,01 à 99 % en poids par rapport à la masse sèche du lyophilisat. Il est également possible de n'introduire que des ballasts et de ne pas employer de matière épaississante.

Les ballasts sont généralement en excès par rapport au pouvoir solubilisant de la pâte à lyophiliser. Ils constituent 1 à 99 % en poids par rapport à la masse sèche totale du lyophilisat. Il est cependant possible de n'introduire que la matière épaississante, sans employer de ballast.

L'agent stabilisant est fonction des particules qui constitueront la pâte à lyophiliser et représente de 1 à 70 % en poids par rapport à la masse sèche lyophilisée.

La quantité d'eau introduite est déterminée de telle manière que la pâte à lyophiliser ait un comportement rhéologique et une viscosité adaptés. Il est en effet souhaitable d'ajuster la viscosité de la pâte à lyophiliser de telle sorte qu'elle soit suffisamment fluide pour permettre une division régulière, et suffisamment visqueuse pour éviter la sédimentation des micro (et/ou nano) particules.

La quantité d'eau introduite dans la constitution du mélange pourra représenter 10 à 90 % en poids par rapport à la masse humide à lyophiliser, en fonction de la nature des substances choisies pour constituer le mélange.

Enfin, le mélange de particules contenant le/les principe(s) actif(s) est préparé de telle manière que le polymère ou la substance macromoléculaire introduite représente 0,1 à 80 % en poids par rapport à la masse sèche du lyophilisat.

En outre, la préparation destinée à être lyophilisée peut contenir éventuellement d'autres additifs comme par exemple des agents de surface, ou d'autres substances compatibles et le cas échéant pharmaceutiquement acceptables telles que des colorants, des substances édulcorantes ou modifiant le goût, des agents conservateurs, ou toute autre substance compatible avec le reste du mélange.

A titre d'exemple, les agents de surface peuvent être choisis parmi les agents non-ioniques [polysorbates polyoxyéthyléniques (Tween), esters de sorbitane (Span), copolymères d'oxyde d'éthylène et de propylène, éthers de polyoxyéthylèneglycols et d'alcool gras ...], les agents anioniques [esters de l'acide sulfosuccinique : sulfosuccinates de dialkyle, par exemple le dioctylsulfosuccinate de sodium], les agents cationiques (sels d'ammoniums quaternaires).

Les substances édulcorantes ou modifiant le goût peuvent être notamment le saccharose, le glucose, le xylose, le sorbitol, la saccharine, les saccharinates, les cyclamates, l'aspartame, le glycyrrhizinate d'ammonium, ou encore les acides citrique, ascorbique ou tartrique, ou tout autre substance habituellement utilisée pour la modification du goût dans l'industrie alimentaire ou pharmaceutique et qui soit compatible avec les produits mis en présence.

Toutes ces substances peuvent être additionnées indifféremment au début, au cours ou à la fin de la constitution de la pâte à lyophiliser.

Il est entendu que cette nouvelle forme unitaire, solide peut s'appliquer à l'administration de toutes sortes de substances et plus spécialement aux principes actifs pharmaceutiques utilisables par voie orale et destinés aussi bien à la médecine humaine qu'à la médecine vétérinaire. Elle s'applique également aux agents de nutrition, aux agents de diagnostic et aux agents cosmétiques.

A titre d'exemple, parmi les substances pharmaceutiquement actives administrables sous cette forme peuvent être cités les anti-infectieux (spiramycine, pristinamycines, tétracyclines, métronidazole, péfloxacine et dérivés de la famille des quinolones, céfixime, josamycine...), les anti-inflammatoires et anti-rhumatismaux (kétoprofène...), les analgésiques (aspirine, paracétamol, clométacine...), les tranquillisants (lorazépam, oxazépam, zopiclone et autres dérivés de la famille de cyclopyrrolones, dérivés de la famille des phénothiazines...), les cardiovasculaires et les vaso-dilatateurs cérébraux (isosorbidedinitrate, trinitrine, dihydroergotoxine, digoxine, quinacaïnol, propranolol, oxprénolol, vincamine, nicergoline...), les protecteurs cérébraux (gangliosides par exemples), les antispasmodiques et antisécrétoires, les antiasthmatiques, les agents thérapeutiques des maladies du tractus gastro-intestinal, les protecteurs hépatiques, les hormones, les contraceptifs, les médicaments destinés au traitement des allergies, les vaccins, les vitamines ou encore des peptides, des polypeptides ou des protéines.

Parmi les agents de nutrition peuvent être cités notamment des acides aminés, par exemple la méthionine, la lysine, le lysinate de carnitine...

La nouvelle forme unitaire, solide s'applique également aux agents de diagnostic in vitro, par exemple l'acridine orange (marqueur de phagocytose), ou aux agents de diagnostic in vivo.

Lorsque la nouvelle forme unitaire, solide s'applique aux agents cosmétiques, le principe actif est notamment choisi parmi les substances modifiant l'haleine comme par exemple le menthol et les essences de menthe ou d'eucalyptus.

La quantité du principe actif introduit est variable en fonction de sa nature mais il est bien entendu que cette nouvelle forme solide peut permettre de préparer des doses unitaires à forte teneur en principe actif, permettant ainsi de diminuer la multiplicité des prises compte tenu de la libération contrôlée de ce dernier. D'une manière générale, la quantité de principe actif peut aller jusqu'à 95 % en poids par rapport à la matière sèche.

La nouvelle forme solide selon l'invention présente ainsi l'avantage d'allier une forme primaire unitaire à désagrégation instantanée en milieu aqueux, à une forme secondaire à dissolution contrôlée constituée d'un système micro (et/ou nano) particulaire.

La forme primaire permet un emploi plus facile, évite notamment l'agglomération des particules et présente surtout l'avantage de contenir une quantité prédéterminée de principe actif.

La forme secondaire contrôle la libération du principe actif au contact des milieux aqueux. Elle s'adresse tout particulièrement aux substances instables en solution. Par exemple lorsque le principe actif est destiné à l'administration par voie orale, la forme de micro (et/ou nano) particules permet de contrôler la mise à disposition du principe actif ; la substance se trouve protégée jusqu'à la zone souhaitée ou jusqu'au moment voulu. La libération de la matière active est alors liée à des facteurs comme le pH, la force ionique, la présence d'une enzyme, la présence d'une flore bactérienne spécifique ou non. Le choix de la dimension des micro (et/ou nano) particules permet de prédéterminer la vitesse de libération du principe actif ; par exemple la vitesse de libération en milieu gastrointestinal lorsque le principe actif est administré par voie orale. Cette forme secondaire peut, bien entendu, contenir plusieurs séries de micro (ou nano) particules soit mélangées les unes aux autres et dans lesquelles les matières actives seront libérées simultanément ou successivement, soit contenues les unes dans les autres et dans lesquelles les matières actives seront libérées successivement. La forme secondaire permet également le masquage du goût de matières actives, par exemple de produits amers.

La nouvelle forme unitaire, solide selon l'invention lorsqu'elle s'applique aux formulations pharmaceutiques est de ce fait tout spécialement indiquée pour l'administration orale, mais elle peut être également utilisée pour l'administration par voie rectale ou vaginale.

Compte tenu des avantages qu'elle présente, la nouvelle forme unitaire, solide selon l'invention est tout spécialement indiquée pour les formulations pharmaceutiques orales destinées à la pédiatrie ou à gériatrie, pour les formulations à bioadhésion buccale comme par exemple les systémes de rafraichissement de l'haleine..., pour les formulations à vectorisation colonique ... ou encore en médecine vétérinaire, dans le cas des adjuvants d'alimentation ou dans le cas des diagnostics médicaux.

Les exemples suivants illustrent la présente invention.

EXEMPLE 1

Préparation d'un lyophilisat contenant des microsphères de spiramycine de 50 à 400 μm de diamètre :

a) 33 g de spiramycine base et 27 g d'Eudragit E 100 sont dissous dans 100 cm3 de dichlorométhane. La solution obtenue est appelée solution A.

b) 14 g d'alcool polyvinylique (Mowiol 8.88 de Hoechst), 3 g de spiramycine base et de 70 g de chlorure de sodium sont dissous dans 700 cm3 d'eau. La solution obtenue est appelée solution B.

c) La solution A est dispersée dans la solution B sous agitation mécanique. L'agitation est maintenue jusqu'à élimination du solvant organique, soit 10 heures à température ambiante.

d) Les microsphères obtenues sont filtrées, lavées et séchées (50 g de matière sèche). On obtient ainsi une poudre jaune pâle dont la saveur est insipide. L'examen au microscope révèle des microparticules individualisées et sphériques de 50 b 400 μm de diamètre. Leur teneur en spiramycine est d'environ 55 %.

e) les microsphères de spiramycine préalablement obtenues sont mélangées à sec avec 46 g de mannitol et 17 g d'oxyde de titane.

f) Un gel fluide est préparé par dissolution de 85 mg de gomme xanthane, 5 g de dextrane 70 et 250 mg de dioctylsulfosuccinate de sodium dans 76 cm3 d'eau.

g) Ce gel fluide est ajouté au mélange des poudres préparé en e) et le mélange est agité sous pression réduite (environ 10 kPa (0,1 bar)]. On obtient une pâte d'environ 685 mPa.s de viscosité relative au gradient de cisaillement 44 $s^{-1}$ (viscosimètre coaxial).

h) La pâte est répartie dans des alvéoles de chlorure de polyvinyle transparent de 1,2 cm3 à raison de 1150 ± 57,5 mg.

i) Dans un lyophilisateur, la pâte est congelée à -25°C puis lyophilisée pendant 5 heures ; la température du produit passe de -25 à +40°C. Les alvéoles sont thermoscellées avec une feuille de complexe aluminum-chlorure de polyvinyle-dichlorure de polyvinyle.

Le lyophilisat obtenu contient 750 000 UI de spiramycine. Sa saveur est insipide. Sa résistance mécanique permet l'extrusion hors de la plaquette thermoformée et la manipulation jusqu'au moment de l'administration.

Le temps de désagrégation du lyophilisat dans l'eau varie de 1 à 3 minutes. Les microsphères ne libèrent pas la spiramycine dans un verre d'eau après 15 minutes ; elles libèrent la spiramycine dans des milieux de pH inférieurs à 5 comme le suc gastrique par exemple.

EXEMPLE 2

On opère comme à l'exemple 1 de a) à d) pour la préparation de microsphères de spiramycine.

e) Les microsphères de spiramycine préalablement obtenues sont mélangées à sec avec 46 g de mannitol, 8,5 g d'oxyde de titane et 8,5 g de carbonate de calcium.

Un gel fluide est préparé comme décrit à l'exemple 1,f) et ajouté au mélange des poudres préparé en e). La pâte obtenue est répartie dans des alvéoles et lyophilisée comme décrit précédemment à l'exemple 1.

EXEMPLE 3

On opère comme à l'exemple 1 de a) à d) pour la préparation de microsphères de spiramycine.

e) Les microsphères de spiramycine préalablement obtenues sont mélangées à sec avec 46 g de mannitol, 8,5 g d'oxyde de titane et 8,5 g de phosphate tricalcique.

Un gel fluide est préparé comme décrit à l'exemple 1,f) et ajouté au mélange des poudres préparé en e). La pâte obtenue est répartie dans des alvéoles et lyophilisée comme décrit précédemment à l'exemple 1.

EXEMPLE 4

On opère comme à l'exemple 1 de a) à d) pour la préparation de microsphères de spiramycine.

e) Les microsphères de spiramycine préalablement obtenues sont mélangées à sec avec 46 g de mannitol, 8,5 g d'oxyde de titane et 8,5 g de kaolin.

Un gel fluide est préparé comme décrit à l'exemple 1,f) et ajouté au mélange des poudres préparé en e). La pâte obtenue est répartie dans des alvéoles et lyophilisée comme décrit précédemment à l'exemple 1.

EXEMPLE 5

On opère comme à l'exemple 1 de a) à d) pour la préparation de microsphères de spiramycine.

e) Les microsphères de spiramycine préalablement obtenues sont mélangées à sec avec 46 g de mannitol, 17 g de phosphate tricalcique.

Un gel fluide est préparé comme décrit à l'exemple 1,f) et ajouté au mélange des poudres préparé en e). La pâte obtenue est répartie dans des alvéoles et lyophilisée comme décrit précédemment à l'exemple 1.

## EXEMPLE 6

On opère comme à l'exemple 1 de a) à d) pour la préparation de microsphères de spiramycine.

e) Les microsphères de spiramycine préalablement obtenues sont mélangées à sec avec 46 g de mannitol, 17 g de kaolin.

Un gel fluide est préparé comme décrit à l'exemple 1,f) et ajouté au mélange des poudres préparé en e). La pâte obtenue est répartie dans des alvéoles et lyophilisée comme décrit précédemment à l'exemple 1.

## EXEMPLE 7

Préparation d'un lyophilisat contenant des microsphères de spiramycine de 50 à 150 µm de diamètre.

a) 66,6 g de spiramycine base et 33,4 g d'éthylcellulose (qualité N 100 de Herculès) sont dissous dans 700 cm3 de dichlorométhane. La solution obtenue est appelée solution A.

b) 45 g d'alcool polyvinylique (Mowiol 8.88 de Hoechst), 9,5 g de spiramycine base et de 225 g de chlorure de sodium sont dissous dans 2100 cm3 d'eau. La solution obtenue est appelée solution B.

c) La solution A est dispersée dans la solution B sous agitation mécanique constante (350 tours/minute) et l'agitation est maintenue jusqu'à élimination du solvant organique, soit 18 heures à température ambiante.

d) Les fractions supérieures à 150 µm et inférieures à 50 µm sont éliminées par tamisage humide. La fraction 50-150 µm est lavée et séchée. On obtient 72,5 g d'une poudre jaune pâle constitués de microsphères individualisées et sphériques de 50 à 150 µm de diamètre. Leur saveur est insipide. Leur teneur en spiramycine est d'environ 66,7 %.

e) les microsphères de spiramycine préalablement obtenues sont mélangées à sec avec 130 g de mannitol et 15 g d'oxyde de titane.

f) Un gel fluide est préparé par dissolution de 40 mg de gomme xanthane, 2,9 g de dextrane 70 et 73 mg de dioctylsulfosuccinate de sodium dans 135 cm3 d'eau.

g) Ce gel fluide est ajouté au mélange des poudres préparé en e) et le mélange est agité sous pression réduite [environ 10 kPa (0,1 bar)].

h) La pâte est répartie dans les alvéoles de chlorure de polyvinyle transparent de 1,2 cm3 à raison de 1200 mg.

i) Dans un lyophilisateur, la pâte est congelée à -25°C puis lyophilisée pendant 5 heures ; la température du produit passe de -25°C à +40°C. Les alvéoles sont thermoscellées avec une feuille de complexe aluminum - chlorure de polyvinyle - dichlorure de polyvinyle.

Le lyophilisat obtenu contient 750 000 UI de spiramycine. Sa saveur est insipide. Sa résistance mécanique permet l'extrusion hors des alvéoles de la plaquette thermoformée et la manipulation jusqu'au moment de l'administration.

Le temps de désagrégation du lyophilisat dans l'eau varie de 15 à 30 secondes. Les microsphères ne libèrent pas la spiramycine dans un verre d'eau après 15 minutes ; elles libèrent la spiramycine dans des milieux de pH inférieurs à 5 comme le suc gastrique par exemple.

## EXEMPLE 8

Préparation d'un lyophilisat contenant des nanosphères d'acridine orange de 100 à 200 nm de diamètre. L'acridine orange est utilisée dans cet exemple à des fins de diagnostic in vitro de phagocytose des nanoparticules par les macrophages.

a) 0,001 g d'acridine orange et 0,5 g de poly (acide D.L lactique) (PLA 50 de masse moléculaire 49 000 de Rhône-Poulenc) sont dissous dans 100 cm3 d'acétone. La solution obtenue est appelée solution A.

b) 0,5 g de polymère mixte d'oxyde d'éthylène et de propylèneglycol (Pluronic F 68® ou Poloxamer 188®) sont dissous dans 200 cm3 d'eau distillée. La solution obtenue est appelée solution B.

c) La solution A est émulsionnée dans la solution B par agitation d'un barreau aimanté à la vitesse de 100 tours par minute.

d) L'agitation est maintenue 24 heures à température ambiante et pression atmosphérique afin d'éliminer l'acétone par évaporation.

e) La suspension obtenue est filtrée sur filtre de 0,8 µm de porosité.

f) On centrifuge à 10 000 tours/minute pendant 1 heure puis on élimine 155 cm3 de surnageant. On obtient ainsi un sédiment de nanosphères d'acridine orange (environ 0,5 g) redispersible dans les 45 cm3 de phase aqueuse restante. L'examen au microscope révèle des nanoparticules individualisées et sphériques de 100 à 300 nm de diamètre. Leur teneur en acridine orange est d'environ 0,2 %.

g) Le sédiment de nanosphères précédemment obtenu est à nouveau dispersé dans les 45 cm3 de phase aqueuse restante. On ajoute 12,5 mg de gomme xanthane (Rhodigel 23 de Rhône-Poulenc) et 1 g de dextrane 70 et on agite jusqu'à obtention d'un gel fluide.

h) 10 g d'oxyde de titane sont mélangés à sec avec 62 g de mannitol.

i) Le gel fluide préparé en g) est ajouté au mélange des poudres préparé en h) et le mélange est agité sous pression réduite [environ 10 kPa (0.1 bar)].

j) La pâte est répartie dans des alvéoles de chlorure de polyvinyle transparent de 1,2 cm3 à raison de 1200 mg.

k) La pâte est congelée à -25°C dans un lyophilisateur puis lyophilisée pendant 5 heures ; la température du produit passe de -25 à +40°C. Les alvéoles sont thermoscellées avec une feuille de complexe aluminium-chlorure de polyvinyle-dichlorure de polyvinyle.

Le lyophilisat obtenu contient environ 0,01 mg d'acridine orange. Sa résistance mécanique permet l'extrusion hors des alvéoles de la plaquette thermoformée et la manipulation jusqu'au moment de l'administration.

Le temps de désagrégation du lyophilisat dans l'eau est inférieur à 1 minute.

EXEMPLE 9

Préparation d'un lyophilisat contenant des microgranules de paracétamol de 0,8 à 1,6 mm de diamètre.

a) 182 g de paracétamol (qualité pulvérisée Rhône-Poulenc) sont mélangés à sec avec 728 g de cellulose microcristalline (qualité Avicel PH 101 de FMC corp.).

b) Le mélange précédent est mouillé avec 6 litres de solution aqueuse à 1,5 % de carboxyméthylcellulose sodique.

c) La masse humide est étrudée sur grille de 1 mm puis sphéronisée pendant 5 minutes environ.

d) On sèche en étuve à 35°C.

e) La fraction de diamètre compris entre 1 et 1,5 mm est sélectionnée par tamisage. On obtient environ 900 g de microgranules sphériques non enrobés de paracétamol de taille comprise entre 1 et 1,5 mm (rendement 90 % environ).

f) Dans un appareil à lit d'air fluidisé, les 1 800 g de la solution ci-dessous sont pulvérisés sur les 900 g de microgranules obtenus en e) :

```
Ethylcellulose dispersion aqueuse à 30 % ............... 50,0 g

(Aquacoat EDC 30 de FMC corp.)

Phtalate de diéthyle ................................. 3,0 g

Oxyde de fer jaune .................................. 0,4 g

Eau distillée ...................................... 46,6 g
```

On obtient environ 1 kg de microgranules à libération prolongée de paracétamol de diamètre compris entre 0,8 et 1,6 mm et de couleur marron. Leur teneur en paracétamol est d'environ 15,2 %.

g) 110 g des microgranules enrobés de paracétamol précédemment obtenus en f) sont mélangés à sec avec 96 g de mannitol et 33 g d'oxyde de titane.

h) Un gel fluide est préparé par dissolution de 500 mg de gomme xanthane (Rhodigel 23 de Rhône-Poulenc), 500 mg de dioctylsulfosuccinate de sodium et 10 g de dextrane 70 dans 150 cm3 d'eau.

i) Ce gel fluide est ajouté au mélange des poudres préparé en g) et le mélange est agité sous pression réduite (environ 0,1 bar).

j) La pâte est répartie dans des alvéoles de chlorure de polyvinyle transparent de 1,2 cm3, à raison de 1200 mg.

k) La pâte est congelée à -25°C dans un lyophilisateur, puis lyophilisée pendant 5 heures ; la température du produit passe de -25 à +40°C. Les alvéoles sont thermoscellées avec une feuille de complexe aluminium - chlorure de polyvinyle - dichlorure de polyvinyle.

Le lyophilisat obtenu contient 50 mg de paracétamol. Sa résistance mécanique permet l'extrusion hors de la plaquette thermoformée et la manipulation jusqu'au moment de l'administration.

Le temps de désagrégation du lyophilisat dans l'eau varie de 1 à 3 minutes. Les microgranules à libération prolongée ne libèrent pratiquement pas de paracétamol au cours de la désagrégation du lyophilisat dans les milieux aqueux.

La libération du paracétamol, à partir des microgranules, est contrôlée par la membrane polymérique.

La cinétique de dissolution in vitro du paracétamol, contrôlée par la méthode n°2 à la palette décrite à l'USP XXI, est représentée par les résultats regroupés dans le tableau ci-joint.

## CINETIQUE DE DISSOLUTION IN VITRO DU PARACETAMOL A PARTIR DE MICROGRANULES ENROBES A LIBERATION PROLONGEE

(Méthode USP XXI à la palette n°2 - 37°C - 120 tours/minute - milieu pH 1 = HCl 0,1 N - milieu pH 7,4 = solution tampon phosphate disodique/acide citrique - Lecture spectrophotomètre UV à 242 nm).

| MILIEU DE DISSOLUTION | TEMPS | POURCENTAGE DISSOUS |
|---|---|---|
| HCl 0,1 N  pH 1 | 30 minutes | 3,5 % |
|  | 1 heure | 5,0 % |
|  | 2 heures | 8,5 % |
| tampon pH 7,4 | 3 heures | 13,5 % |
|  | 4 heures | 17,5 % |
|  | 5 heures | 32,0 % |
|  | 6 heures | 50,5 % |
|  | 7 heures | 65,0 % |
|  | 8 heures | 77,5 % |
|  | 24 heures | 100,0 % |

## EXEMPLE 10

Préparation d'un lyophilisat oral contenant des microsphères de kétoprofène.

a) 60 g de kétoprofène et 240 g d'un mélange éthylcellulose/Eudragit RS 100 (9 : 1) sont dissous dans 1,5 litre de dichlorométhane à l'aide d'un agitateur à pâle (Heidolph) à 500 tours/mn,

b) à la solution précédente, 1,5 litre d'une solution aqueuse de Méthocel K4M à 0,27 % (poids/volume) est additionnée pour obtenir une émulsion,

c) après évaporation du dichlorométhane sous pression réduite les microsphères sont séparées par centrifugation et lavées par 3 litres d'eau,

Les microsphères sont séchées à température ambiante pendant 2 à 3 heures dans un appareil à lit d'air fluidisé,

9

d) 500 mg de microsphères obtenues comme décrit ci-dessus (dosées à 20,2 % en principe actif, sont mélangés à sec avec :

```
gomme xanthane (Rhodigel 23 de RHONE-POULENC) .....      0,75 mg

dioctylsulfosuccinate de sodium ..................      0,38 mg

Dextrane 70 ......................................     30,00 mg

Lactose ..........................................    218,87 mg
```

puis additionnés de 500 mg d'eau
e) la pâte est répartie dans des alvéoles de chlorure de polyvinyle, puis lyophilisée dans les conditions décrites aux exemples précédents.
Le lyophilisat obtenu contient 100 mg de kétoprofène.
Le temps de désagrégation du lyophilisat est de 1mn 20s

## Revendications

**Revendications pour les Etats contractants suivants : AT, BE, CH, DE, FR, GB, IT, LI, LU, NL, SE**

1. Une composition caractérisée en ce qu'il s'agit d'une forme unitaire, solide et poreuse susceptible d'être obtenue par :
   1) préparation d'un mélange de micro (et/ou nano) particules, respectivement d'un diamètre de 1 $\mu$m à 2 mm (ou inférieur à 1 $\mu$m) contenant une quantité prédéterminée d'un ou plusieurs principes actifs,
   2) incorporation du mélange obtenu dans une pâte destinée à être lyophilisée et contenant
      a) au moins une substance choisie parmi
         - des matiéres épaississantes et structurantes servant de support et
         - des ballasts,
      b) un ou plusieurs agents stabilisants empêchant la décantation et/ou la remontée en surface des micro (et/ou nano) particules dans le mélange, choisis parmi des substances particulaires de taille micronique, inertes vis à vis du mélange et dont la vitesse de sédimentation est du même ordre de grandeur que celle des particules,
      c) éventuellement un ou plusieurs autres principes actifs ou mélanges de micro (et/ou nano) particules contenant un principe actif,
      d) une quantité d'eau convenable de manière à ajuster la viscosité de la composition,
   3) lyophilisation de la pâte obtenue, éventuellement après répartition dans des alvéoles de forme et de dimension prédéterminées,
   4) éventuellement division du lyophilisat en doses unitaires de forme et de volume prédéterminés.

2. Une composition selon la revendication 1, caractérisée en ce qu'elle s'applique à une administration par voie orale.

3. Une composition selon la revendication 1, caractérisée en ce qu'elle s'applique à une administration par voie rectale ou vaginale.

4. Une composition selon la revendication 1, caractérisée en ce que le principe actif est une substance thérapeutiquement active.

5. Une composition selon la revendication 1, caractérisée en ce que le principe actif est un agent de nutrition, un agent de diagnostic ou un agent cosmétique.

6. Une composition selon la revendication 1, caractérisée en ce qu'elle s'applique au domaine vétérinaire.

7. Un procédé de préparation d'une forme unitaire, solide et poreuse selon la revendication 1 caractérisé en ce que l'on
   1) prépare un mélange de micro (et/ou nano) particules respectivement d'un diamètre de 1 $\mu$m à 2 mm (ou inférieur à 1 $\mu$m) contenant une quantité prédéterminée d'un ou plusieurs principes actifs,

2) incorpore le mélange obtenu dans une pâte destinée à être lyophilisée et contenant

a) au moins une substance choisie parmi

- des matières épaississantes et structurantes servant de support et
- des ballasts,

b) un ou plusieurs agents stabilisants empêchant la décantation et/ou la remontée en surface des micro (et/ou nano) particules dans le mélange, choisis parmi des substances particulaires de taille micronique, inertes vis à vis du mélange et dont la vitesse de sédimentation est du même ordre de grandeur que celle des particules,

c) éventuellement un ou plusieurs autres principes actifs ou mélanges de micro (et/ou nano) particules contenant un principe actif,

d) une quantité d'eau convenable de manière à ajuster la viscosité de la composition,

3) lyophilise la pâte obtenue, éventuellement après l'avoir répartie dans des alvéoles de forme et de dimension prédéterminées,

4) éventuellement divise le lyophilisat en doses unitaires de forme et de volume prédéterminés.

**Revendication pour les Etats contractants suivants : ES, GR**

1. Un procédé de préparation d'une forme unitaire, solide et poreuse caractérisé en ce que l'on

1) prépare un mélange de micro (et/ou nano) particules respectivement d'un diamètre de 1 µm à 2 mm (ou inférieur à 1 µm) contenant une quantité prédéterminée d'un ou plusieurs principes actifs,

2) incorpore le mélange obtenu dans une pâte destinée à être lyophilisée et contenant

a) au moins une substance choisie parmi

- des matières épaississantes et structurantes servant de support et
- des ballasts,

b) un ou plusieurs agents stabilisants empêchant la décantation et/ou la remontée en surface des micro (et/ou nano) particules dans le mélange, choisis parmi des substances particulaires de taille micronique, inertes vis à vis du mélange et dont la vitesse de sédimentation est du même ordre de grandeur que celle des particules,

c) éventuellement un ou plusieurs autres principes actifs ou mélanges de micro (et/ou nano) particules contenant un principe actif,

d) une quantité d'eau convenable de manière à ajuster la viscosité de la composition,

3) lyophilise la pâte obtenue, éventuellement après l'avoir répartie dans des alvéoles de forme et de dimension prédéterminées,

4) éventuellement divise le lyophilisat en doses unitaires de forme et de volume prédéterminés.

**Patentansprüche**

**Patentansprüche für folgende Vertragsstaaten : AT, BE, CH, DE, FR, GB, IT, LI, LU, NL, SE**

1. Zusammensetzung, dadurch gekennzeichnet, daß es sich um eine feste und poröse Einheitsform handelt, erhältlich durch

(1) Herstellung eines Gemisches von Mikro (und/ oder Nano ) teilchen bzw. Teilchen mit einem Durchmesser von 1 µm bis 2 mm (oder niedriger als 1 µm), enthaltend eine vorherbestimmte Menge eines oder mehrerer Wirkstoffe,

(2) Einbrigen des erhaltenen Gemisches in eine für die Lyophilisierung vorgesehene Paste, enthaltend

(a) zumindest eine Substanz, ausgewählt unter

- als Träger dienenden, verdickenden und Struktur verleihenden Materialien und
- Ballaststoffen,

(b) ein oder mehrere Stabilisierungsmittel, die die Dekantation und/oder das Wiederaufsteigen an die Oberfläche der Mikro(und/oder Nano)teilchen in dem Gemisch verhindern, ausgewählt unter teilchenförmigen Substanzen mikronischer Größe, die gegenüber dem Gemisch inert sind und deren Sedlmentationsgeschwindigkeit in der gleichen Größenordnung liegt wie diejenige der Teilchen,

(c) gegebenenfalls einen oder mehrere Wirkstoffe oder Gemische von einen Wirkstoff enthaltenden Mikro(und/oder Nano)teilchen,

(d) eine für die Einstellung der Viskosität der Zusammensetzung geeignete Wassermenge,

(3) Lyophilisierung der erhaltenen Paste, gegebenenfalls nach Verteilung in Hülsen mit vorherbestimmter Form und Dimension,

(4) gegebenefalls Aufteilung des Lyophilisats in Einheitsdosen mit vorherbestimmter Form und vorherbestimmtem Volumen.

2. Zusammensetzung gemäß Anspruch 1, dadurch gekennzeichnet, daß sie sich für eine orale Verabreichung anwenden läßt.

3. Zusammensetzung gemäß Anspruch 1, dadurch gekennzeichnet, daß sie sich für eine rektale oder vaginale Verabreichung anwenden läßt.

4. Zusammensetzung gemäß Anspruch 1, dadurch gekennzeichnet, daß der Wirkstoff eine therapeutisch wirksame Substanz ist.

5. Zusammensetzung gemäß Anspruch 1, dadurch gekennzeichnet, daß der Wirkstoff ein Nährmittel, ein diagnostisches Mittel oder ein kosmetisches Mittel ist.

6. Zusammensetzung gemäß Anspruch 1, dadurch gekennzeichnet, daß sie sich auf veterinärmedizinischem Gebiet anwenden läßt.

7. Verfahren zur Herstellung einer festen und porösen Einheitsform gemäß Anspruch 1, dadurch gekennzeichnet, daß man

(1) ein Gemisch von Mikro(und/oder Nano)teilchen bzw. Teilchen mit einem Durchmesser von 1 µm bis 2 mm (oder geringer als 1 µm), enthaltend eine vorherbestimmte Menge eines oder mehrerer Wirkstoffe, herstellt,

(2) das erhaltene Gemisch in eine für die Lyophilisierung vorgesehene Paste einbringt, die enthält

(a) zumindest eine Substanz, ausgewählt unter

- als Träger dienenden, verdickenden und Struktur verleihenden Materialien und
- Ballaststoffen,

(b) ein oder mehrere Stabilisierungsmittel, die die Dekantation und/oder das Wiederaufsteigen an die Oberfläche der Mikro(und/oder Nano)teilchen in dem Gemisch verhindern, ausgewählt unter teilchenförmigen Substanzen mikronischer Größe, die gegenüber dem Gemisch inert sind und deren Sedimentationsgeschwindigkeit in der gleichen Größenordnung liegt wie diejenige der Teilchen,

(c) gegebenenfalls einen oder mehrere weitere Wirkstoffe oder Gemische von Mikro(und/oder Nano)teilchen, enthaltend einen Wirkstoff,

(d) eine für die Einstellung der Viskosität der Zusammensetzung geeignete Wassermenge,

(3) die erhaltene Paste gegebenefalls nach Verteilung in Hülsen mit vorherbestimmter Form und Dimension lyophilisiert,

(4) gegebenenfalls das Lyophilisat in Einheitsdosen mit vorherbestimmter Form und vorherbestimmtem Volumen aufteilt.

## Patentanspruch für folgende Vertragsstaaten : ES, GR

1. Verfahren zur Herstellung einer festen und porösen Einheitsform, dadurch gekennzeichnet, daß man

(1) ein Gemisch von Mikro(und/oder Nano)teilchen bzw. Teilchen mit einem Durchmesser von 1 µm bis 2 mm (oder geringer als 1 µm), enthaltend eine vorherbestimmte Menge eines oder mehrerer Wirkstoffe, herstellt,

(2) das erhaltene Gemisch in eine für die Lyophilisierung vorgesehene Paste einbringt, die enthält

(a) zumindest eine Substanz, ausgewählt unter

- als Träger dienenden, verdickenden und Struktur verleihenden Materialien und
- Ballaststoffen,

(b) ein oder mehrere Stabilisierungsmittel, die die Dekantation und/oder das Wiederaufsteigen an die Oberfläche der Mikro(und/oder Nano)teilchen in dem Gemisch verhindern, ausgewählt unter teilchenförmigen Substanzen mikronischer Größe, die gegenüber dem Gemisch inert sind und deren Sedimentationsgeschwindigkeit in der gleichen Größenordnung liegt wie diejenige der Teilchen,

(c) gegebenenfalls einen oder mehrere weitere Wirkstoffe oder Gemische von Mikro(und/oder Nano)teilchen, enthaltend einen Wirkstoff,

(d) eine für die Einstellung der Viskosität der Zusammensetzung geeignete Wassermenge,

(3) die erhaltene Paste gegebenenfalls nach Verteilung in Hülsen mit vorherbestimmter Form und Dimension lyophilisiert,

(4) gegebenenfalls das Lyophilisat in Einheitsdosen mit vorherbestimmter Form und vorherbestimmten Volumen aufteilt.

## Claims

**Claims for the following Contracting States : AT, BE, CH, DE, FR, GB, IT, LI, LU, NL, SE**

1.  A composition, characterised in that it is a solid and porous unitary form capable of being obtained by:
    1) preparation of a mixture of micro(and/or nano)particles 1 μm to 2 mm (or less than 1 μm), respectively, in diameter, containing a predetermined quantity of one or more active principles,
    2) incorporation of the mixture obtained into a paste designed to be lyophilised and containing
        a) at least one substance chosen from
            - thickening and structuring materials acting as a carrier, and
            - ballasts,
        b) one or more stabilising agents preventing the micro(and/or nano)particles in the mixture from settling and/or rising to the surface, chosen from micron-sized particulate substances which are inert with respect to the mixture and whose sedimentation rate is of the same order of magnitude as that of the particles,
        c) optionally, one or more other active principles or mixtures of micro(and/or nano)particles containing an active principle,
        d) a suitable quantity of water so as to adjust the viscosity of the composition,
    3) lyophilisation of the paste obtained, optionally after distribution in cells of predetermined shape and size,
    4) optionally, division of the lyophilisate into single doses of predetermined shape and volume.

2.  A composition according to Claim 1, characterised in that it is used in oral administration.

3.  A composition according to Claim 1, characterised in that it is used in rectal or vaginal administration.

4.  A composition according to Claim 1, characterised in that the active principle is a therapeutically active substance.

5.  A composition according to Claim 1, characterised in that the active principle is a nutritional agent, a diagnostic agent or a cosmetic agent.

6.  A composition according to Claim 1, characterised in that it is used in the veterinary field.

7.  A process for preparing a solid and porous unitary form according to Claim 1, characterised in that
    1) a mixture of micro(and/or nano)particles 1 μm to 2 mm (or less than 1 μm), respectively, in diameter, containing a predetermined quantity of one or more active principles, is prepared,
    2) the mixture obtained is incorporated in a paste designed to be lyophilised and containing
        a) at least one substance chosen from
            - thickening and structuring materials acting as a carrier, and
            - ballasts,
        b) one or more stabilising agents preventing the micro(and/or nano)particles in the mixture from settling and/or rising to the surface, chosen from micron-sized particulate substances which are inert with respect to the mixture and whose sedimentation rate is of the same order of magnitude as that of the particles,
        c) optionally, one or more other active principles or mixtures of micro(and/or nano)particles containing an active principle,
        d) a suitable quantity of water so as to adjust the viscosity of the composition,
    3) the paste obtained is lyophilised, optionally after being distributed in cells of predetermined shape and size,
    4) the lyophilisate is optionally divided into single doses of predetermined shape and volume.

## Claim for the following Contracting States : ES, GR

1.  A process for preparing a solid and porous unitary form, characterised in that
    1) a mixture of micro(and/or nano)particles 1 μm to 2 mm (or less than 1 μm), respectively, in diameter,

13

containing a predetermined quantity of one or more active principles, is prepared,

2) the mixture obtained is incorporated in a paste designed to be lyophilised and containing

    a) at least one substance chosen from

       - thickening and structuring materials acting as a carrier, and

       - ballasts,

    b) one or more stabilising agents preventing the micro(and/or nano)particles in the mixture from settling and/or rising to the surface, chosen from micron-sized particulate substances which are inert with respect to the mixture and whose sedimentation rate is of the same order of magnitude as that of the particles,

    c) optionally, one or more other active principles or mixtures of micro(and/or nano)particles containing an active principle,

    d) a suitable quantity of water as as to adjust the viscosity of the composition,

3) the paste obtained is lyophilised, optionally after being distributed in cells of predetermined shape and size,

4) the lyophilisate is optionally divided into single doses of predetermined shape and volume.